Europäisches Patentamt

European Patent Office

Office européen des brevets

(11) Publication number : **0 629 350 A1**

(12) **EUROPEAN PATENT APPLICATION**

(21) Application number : 94810352.8

(22) Date of filing : 14.06.94

(51) Int. Cl.$^5$ : **A23J 3/34**, A23L 1/305

(30) Priority : **16.06.93 GB 9312369**

(43) Date of publication of application :
**21.12.94 Bulletin 94/51**

(84) Designated Contracting States :
**AT BE CH DE DK ES FR GB GR IE IT LI LU NL PT SE**

(71) Applicant : **SANDOZ NUTRITION LTD.**
**Monbijoustrasse 118**
**CH-3001 Berne (CH)**

(72) Inventor : **Belli, Dominique C.A.**
**322 Rte Hermance**
**CH-1247 Anières,Geneva (CH)**
Inventor : **Eigenmann, Philippe**
**6801 Harrowdale Road**
**Baltimore, MD21209 (US)**
Inventor : **Kahn, Jean-Maurice**
**Junkerngasse 25**
**CH-3011 Berne (CH)**
Inventor : **Polla, Barbara**
**18-20 Place Cornavin**
**CH-1201 Geneva (CH)**

(74) Representative : **Smolders, Walter et al**
**Sandoz Technology LTD.,**
**Patents and Trademarks Division,**
**Lichtstrasse 35**
**CH-4002 Basle (CH)**

(54) **Milk protein hydrolysates.**

(57) The use of cow's milk protein hydrolysate substantially free of allergenic proteins to induce cow's milk protein tolerance in children susceptible to cow's milk allergy and in the prophylaxis or treatment of type 1 diabetes mellitus in children susceptible to such disease.

Figure 1

Fig. 1a  Fig. 1b

Fig. 1c  Fig. 1d

EP 0 629 350 A1

Figure 2

## Fig. 2a

## Fig. 2b

The invention relates to milk protein hydrolysates and their use to induce immunologic tolerance in infants suffering from cow's milk allergy.

EPA 421 309 (Sandoz Nutrition Ltd.) discloses non-allergenic cow milk protein hydrolysates having a high degree of hydrolysis whilst having a low content of free amino acids. The non-allergenic milk protein hydrolysates disclosed in EPA 421 309 are indicated for use as protein supply replacement in infants having intolerance against dietary proteins such as cow's milk proteins.

The terms non-allergenic hydrolysates and hydrolysates substantially free of allergenic proteins as used herein are interchangeable. They refer to protein hydrolysates that can be administered to infants having intolerance against dietary proteins, more particularly cow's milk proteins, without inducing allergic reactions.

In allergic disease, the type of allergy is related with the age to the child as food allergy mostly occur in infants. Cow's milk allergy is a common food allergy in infants, the disease prevalence ranging from 2 to 7,5 % of all children. Various symptoms can occur. Symptoms can be immediate (e.g. anaphylaxis and urticaria) which occur in half of the patients or less specific (e.g. vomiting or diarrhoea) which represent delayed clinical reactions in the other half of patients. Diagnostic procedure is well standardized with the criteria given by the European Society for Paediatric Gastroenterology and Nutrition Working Group (ESPGAN J. Pediatr. Gastroenterol. Nutr. 1992; 14: 108-12). Complete exclusion of cow's milk proteins and diet with hydrolysed formulas represent the treatment of choice of this condition. Cow's milk allergy disappears spontaneously. Cow's milk tolerance tends to appear at 2 to 4 years of age, whereby it is been suggested for safety reasons to avoid administration of milk proteins to children suffering from cow's milk allergy before they are 6 to 7 years old.

It has now surprisingly been found that non-allergenic whey protein hydrolysates are capable of inducing cow's milk protein tolerance such that cow's milk allergy disappears substantially faster than normally would be expected for spontaneously (i.e. naturally) acquired tolerance.

The invention accordingly provides a method of inducing cow's milk protein tolerance in children susceptible to cow's milk allergy by administering to a child in need of such treatment a whey protein hydrolysate substantially free of allergenic proteins.

The whey protein hydrolysate free of allergenic proteins is conveniently administered in the form of a complete formula diet.

Another embodiment of the invention is the use of whey protein hydrolysate substantially free of allergenic proteins for the manufacture of a dietary composition for the induction of cow's milk protein tolerance.

Examples of non-allergenic whey protein hydrolysates suitable for use in the method of the invention are hydrolysates obtainable from whey proteins enzymatically hydrolysed by trypsin-chymotrypsin in the presence of a cationic serine endoprotease of the elastase 2 type, e.g. at a temperature of from 35°C to 50°C and a pH between 7.0 and 9.0

The whey protein fraction employed as starting material will preferably be substantially free of macrolipids; i.a. to facilitate enzymatic hydrolysis.

The whey proteins may have been pretreated, e.g. subjected to microfiltration/ultrafiltration, to remove most of large proteins having a molecular weight of more than 60'000 Daltons. Such pretreated whey protein mixture is hereinafter designated "selected whey protein". It will still contain some bovine serum albumine (BSA) having a molecular weight of ca. 66.000 Da.

The use of elastase 2 allows the elimination of allergenic epitopes and provides a product having a high effective degree of hydrolysis (19 % or more), a low content of free amino acids while having a high content of di- to decapeptides and in particular of tetra- to decapeptides.

Preferably the whey protein, more preferably the selected whey protein, is initially subjected to a gastric phase prehydrolysis, e.g. by heating a solution of (selected) whey protein in water to 43 ± 4°C and treating said solution with pepsin at pH between 2.0 and 3.0.

Pepsin hydrolysis of BSA results for example in very little small peptides but facilitates by denaturation the subsequent elimination of peptide fragments of high molecular weight (30.000 to 40.000 Da) by treatment with other enzymes. Elastase 2 allows i.a. the reduction of peptides having an intermediate size (ca. 10.000 Da) and induces a shift of the peptide profile in favour of low molecular weight peptides.

Accordingly, the non-allergenic whey protein hydrolysates particularly suitable for use in the method of the invention are obtainable by physiological hydrolysis of selected whey protein. Said physiological hydrolysis involves a gastric phase, i.e. a pepsin prehydrolysis at pH between 2.0 and 3.0, followed by an enzymatic treatment of the prehydrolysate with a mixture of trypsin-chymotrypsin and a cationic serine endoprotease type elastase 2. Suitable conditions for such physiological hydrolysis are disclosed in EPA 421 309.

The effective degree of hydrolysis of non-allergenic protein hydrolysates obtainable by physiological hydrolysis will conveniently be in the range of from 20 % to 32 %. The free amino acid content of whey protein hydrolysates suitable for use in the method of the invention, is preferably below 15 %, more preferably in the range of from 1 to 13 % by weight.

The effective degree of hydrolysis is determined according to the formula:

$$\frac{\text{g of N in free NH}_2 \text{ groups}}{\text{g of total N (NH, NH}_2)} \times 100$$

A typical molecular weight distribution of non-allergenic whey protein hydrolysates obtainable by physiological hydrolysis suitable for use in the method of this invention, is as follows (determined by UV detection at 215 nm);

|  | % by weight from | more preferably | e.g. |
|---|---|---|---|
| MW > 5000 | 0.5 to 4.0 | 2.8 to 3.8 | 3.3 |
| 5000 > MW > 3000 | 2 to 10 | 5 to 10 | 5.2 |
| 3000 > MW > 1000 | 29 to 36 | 33.5 to 35.5 | 33.9 |
| 1000 > MW > 500 | 26 to 31 | 29 to 31 | 30.9 |
| 500 > MW > 300 | 14.6 to 29.6 | 15 to 21 | 20.6 |
| 300 > MW | 5.9 to 12.9 | 5.7 to 7.7 | 6.1 |

Such non-allergenic whey protein hydrolysates are preferably administered in admixture with non-allergenic casein hydrolysates in a weight ratio whey protein hydrolysate: casein protein hydrolysate of 50:50 to 70:30, preferably 60:40, such that its composition in g amino acid per 100 g hydrolysate is similar to that of mother's milk.

Non-allergenic casein protein hydrolysates are obtainable by enzymatic treatment of casein with a mixture of trypsin-chymotrypsin and a cationic serine endoprotease type elastase 2. Casein hydrolysates are in general less problematic than whey protein hydrolysates from the allergenicity point of view. Thus it is in general not necessary to subject casein - in addition to the aforementioned enzymatic treatment - to a gastric phase hydrolysis.

Typically , the casein used as starting material for preparation of casein hydrolysates suitable for use in the method of the invention, will be casein substantially free of glycoprotein fraction; the casein employed is conveniently rennet casein, i.e. as obtained by coagulation with pepsin, e.g. bovine pepsin.

A typical molecular weight distribution of non-allergenic casein hydrolysates suitable for use in admixture with non-allergenic whey protein hydrolysates in the method of the invention is as follows (determined by UV detection at 215 nm):

|  | % by weight range | e.g. |
|---|---|---|
| MW > 5000 | 0 to 1 | 0 |
| 5000 > MW > 3000 | 0 to 1 | 0.1 |
| 3000 > MW > 1000 | 17 to 26 | 18.6 |
| 1000 > MW > 500 | 33 to 40 | 37.4 |
| 500 > MW > 300 | 26 to 31 | 29.2 |
| 300 > MW | 11 to 16 | 14.7 |

The effective degree of hydrolysis of particularly suitable casein hydrolysates lies in the range of from 20 to 30 %.

The free amino acid content in the casein hydrolysates suitable for use in the invention is preferably below 15 % and more preferably in the range of from 8 to 13 %.

The whey protein hydrolysates and casein protein hydrolysates may be treated with a proteolytic enzyme from the fungus Aspergillus oryzae or with a pancreatic proteinase extract for taste improvement. Such enzymes are commercially available. Typical examples thereof include COROLASE 7092 and COROLASE PP (Röhm).

The treatment of milk proteins with a COROLASE 7092 type of proteolytic enzyme is conveniently at a pH of about 8 and employing ca. 1.0 to 1.5 % enzyme/protein substrate having an activity of about 2000 m UHb/mg enzyme (see Example 4 hereinafter) during ca. $1\tfrac{1}{4}$ to $1\tfrac{3}{4}$ hours.

The proteolytic enzyme from Aspergillus oryzae is conveniently added to the protein mixture comprising an effective amount of pancreatic enzymes (trypsin-chymotrypsin and optionally elastase 2), preferably after 1 hr to $1\tfrac{1}{2}$ hr treatment with pancreatic enzymes at 41 to 47° C.

Treatment with 1.0 to 1.5 % COROLASE PP type enzyme/protein substrate at a pH of about 8 during $1\tfrac{1}{4}$ to $1\tfrac{3}{4}$ hours at 41 to 47° C allows similar taste improvement.

The preparation of the starting materials, the hydrolysis conditions and working up of the hydrolysates including any pasteurization, ultrafiltration, concentration and drying steps may be carried out in a manner known per se, e.g. analogous to the methods disclosed in EPA 421 309 or as exemplified hereinafter.

It will be appreciated that the composition of the hydrolysates obtained by hydrolysis of milk proteins or milk protein fractions will depend on the hydrolysis conditions.

Preferably the conditions will be selected such that the hydrolysate has an effective degree of hydrolysis and a free amino acid content within the ranges specified herein.

In general it will be preferred to use a non-allergenic whey protein hydrolysate, more preferably a whey protein hydrolysate: casein hydrolysate mixture in a weight ratio in the range of from 5:1 to 1:1, most preferably 1.5:1.

The whey protein hydrolysate fraction to be employed comprises conveniently less than 35 % by weight preferably from 20 to 33 % by weight of peptides containing 2 to 5 amino acids and/or from 35 to 60 % by weight of peptides having 4 to 10 amino acids when determined by UV detection at 215 nm. (It will be appreciated that the values will vary within said ranges to some extent due to natural variation of the composition of the starting material, but also depending on the detection method employed: the chromatographic analysis by UV 215 nm detection and by Refraction Index detection will give different results).

The ability of milk protein hydrolysates to induce cow's milk protein tolerance is indicated by results obtained employing the Lymphocyte stimulation test (LST).

## Background

Lymphocyte stimulation test (LST) was performed with sterile blood of a cow's milk allergic child in order to study cellular response to the offending proteins before, and after 3 months exclusion diet. The child developed during the treatment with DAMIRA® (Sandoz Nutrition Ltd.) a cellular tolerance showed by cellular stimulation only be higher concentration of the offending proteins.

The mild protein hydrolysate used as test mixture is a commercially available product, designated DAMIRA® (Sandoz Nutriton Ltd.).

DAMIRA® comprises a hydrolysate from 60 % whey protein and 40 % rennet casein. The whey protein hydrolysate has been obtained by physiological hydrolysis of whey protein, substantially free of macrolipids and lactose, i.e. involving a gastric phase (pepsin hydrolysis) and a pancreatic hydrolysis (with trypsin/chymotrypsin and elastase 2), essentially as disclosed in Example 1 hereinafter. The rennet casein hydrolysate has been obtained essentially as disclosed in Example 7 hereinafter.The DAMIRA® composition is given in Example 8.

## Test Description

### Lymphocyte stimulation test

This test is performed on the lymphomonocyte fraction isolated from sterile heparinized blood after centrifugation on Ficoll (Pharmacia). The cells are suspended on a buffer solution (TC 199 Hanks), centrifugated and washed 3 times. They are then added to a solution of AB human serum (at a concentration of 20 %) with culture medium (RPMI 1640). $10^6$ cells/ml are incubated in quaduplicates in 96 well plates with the antigens at the different concentrations. Six different logarithmic concentrations (from $10^{-8}$ to $10^{-3}$ mg/ml of whole milk, casein, α-lactalbumin, β-lactoglobulin and a milk protein hydrolysate product are studied. After incubation for 7 days, the cell DNA is labelled with methyl-H3 thymidine; collected on a paper filter and mixed with a scintillation liquid. Radioactivity expressing the cell proliferation is measured on a Beta counter. The results are expressed either as total counts per minute (cpm) or as stimulation index (SI):

$$\frac{\text{cpm with antigen}}{\text{cpm in negative controls}}.$$

An index > 3 is interpreted as positive. Negative controls without antigen; phytohemagglutinin and tetanus toxoid are used as positive controls.

## Results

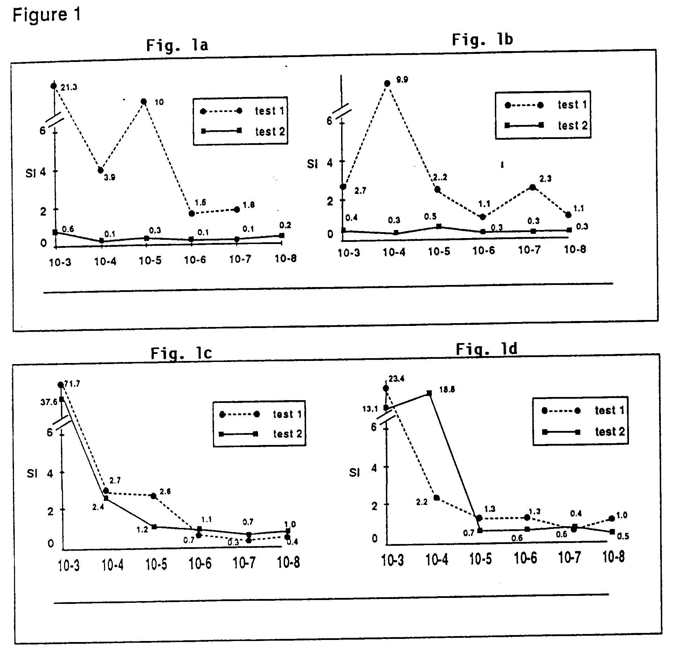

Figure 1 represents the cellular response to whole cow milk and the 3 major antigens at diagnosis (test 1) and after 3 months (test 2) treatment with DAMIRA®. Fig. 1a gives the Stimulation Index (SI) for whole milk concentrations, Fig. 1b for $\alpha$-lactalbumin concentrations. Fig. 1c for $\beta$-lactoglobulin concentrations and Fig. 1d for casein concentrations.

Stimulation indexes are higher for whole cow milk at all the concentrations at the time of test 1 as compared to test 2.This tendency is also observed for alpha-lactalbumin and $\beta$-lactoglobulin. The index was positive until 10-5 mg/ml at test 1 and negative at all concentrations at test 2 for whole cow milk. It was negative at 10-4 mg/ml for $\beta$-lactoglobulin at both tests; positive at 10-4 at test 1 and negative for all concentrations at test 2 for $\alpha$-lactoalbumin and for casein positive at 10-4 at test 2 and negative at the same concentration at test 1.

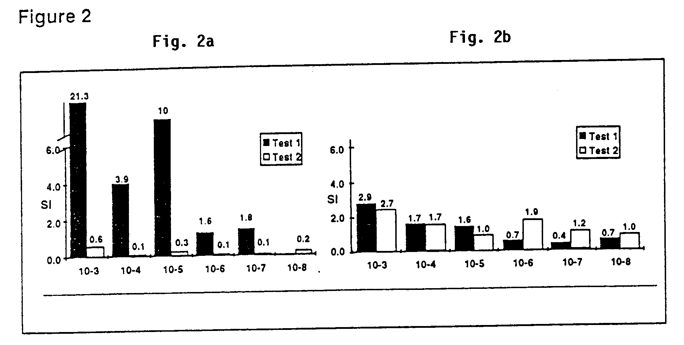

Figure 2 compares whole cow milk (Fig. 2a) and DAMIRA® (Fib. 2b) at 3 months interval. Stimulation indexes were positive up to 10-5 mg/ml for milk at test 1 whereas stimulation indexes for DAMIRA® were negative at all the concentrations at test 1. At test 2, stimulation indexes were negative at all concentrations for both. In contrast, lymphocyte proliferation was less with whole cow milk than with DAMIRA® at all concentrations tested. These results suggest the apparition of an immunologic tolerance during the diet.

The positive results with DAMIRA® are confirmed in further tests.

In view of its tolerance inducing effect, the protein hydrolysates are also indicated for prophylaxis and therapy of infants and children having a genetic predisposition to type I diabetes mellitus:

It has indeed been suggested that Bovine Serum Albumin (BSA) triggers the immune system. Anti-BSA antibodies, in particular IgG anti-BSA antibodies bind to the endogenous antigen p69 which is located on the surface of pancreatic $\beta$-cells and has a chemical structure similar to the portion of the BSA peptide triggering in allergic reaction. Such immune attack reduces the $\beta$-cell population leading to the development of type I diabetes millitus.

Administration of the milk protein hydrolysate disclosed herein prevents destruction of pancreatic beta cells in susceptible persons. Timely diagnosis of genetic predisposition to type I diabetes mellitus will accordingly allow prophylaxis and therapy of type I diabetes mellitus in susceptible infants and children by treatment with protein hydrolysates. The treatment will conveniently be continued till the children acquired cow milk tolerance.

For use in the methods of the invention, the protein hydrolysates according to the invention are conveniently administered in nutritionally acceptable composition form. Such compositions may comprise carbohydrate and fatty acid sources, vitamins, minerals and trace elements.

Said compositions are preferably in the form of a complete formula diet (in liquid or powder form), such that, when used as sole nutrition source essentially all daily caloric, nitrogen, fatty acid, vitamin, mineral and trace element requirements are met.

For infants, the daily caloric amount to be supplied will in general lie in the range of from 100 to 180 Kcal per kg body weight. The contribution of the nitrogen source (i.e. the whey/casein hydrolysate of the invention), carbohydrate source and lipid source to the total daily amount may vary within wide ranges. In typical compositions of the invention, the carbohydrate source provides for 45 to 68 %, the fatty acid sources for 25 to 50 % and the protein hydrolysate of the invention for 7 to 15 % of the total energy supply of the composition.

An example of carbohydrates particularly suitable for use in the complete diet for infants includes a mixture on the basis of maltodextrines (10 to 25 %) and lactose (90 to 75 %), unless the infant requires a diet having a low lactose content, in which case the carbohydrate source will conveniently be quasi exempt of lactose (< 1 % lactose).

Examples of suitable fatty acid sources include triglyceride oils and phospholipids.

The carbohydrates employed for composition for adults are preferably primarily a mixture on the basis of maltodextrines having a low mono- and disaccharide content (< 5 % by weight of the total carbohydrate content), a very low content of alimentary fibers and being quasi exempt of lactose. Preferably such compositions will have a total lactose content of less than 1 % by weight of the protein hydrolysate present in the formulation.

Examples of vitamins suitable for incorporation in the composition of the invention include vitamin A, vitamin $D_3$, vitamin E, vitamin $k_1$, vitamin C, folic acid, thiamin, riboflavin, vitamin $B_6$, vitamin $B_{12}$, niacinamid, biotin, l-carnitine, choline and panthotenic acid in physiologically acceptable form. Depending on the contemplated use, the incorporation of taurine and/or hypotaurine, of L-cystine, resp. supplementation of threonine, may be useful. The amount of amino acids added to the composition of the invention, in free amino acid form or in small peptide form, will preferably be such that it does not substantially influence the composition of the non-allergenic hydrolysates employed in the method of the invention e.g. in terms of free amino acid content, effective degree of hydrolysis, content of peptides having 4 to 10 amino acids and the like.

Examples of minerals and trace elements suitable for incorporation in the composition of the invention include sodium, potassium, calcium, phosphorous, magnesium, manganese, copper, zinc, iron, selenium, chromium and molybdenum in physiologically acceptable form.

It will be appreciated that the minimum daily requirements of vitamins, minerals and trace elements will depend on the person to be treated. In general, the daily minimum requirements are determined by governmental authorities; they may accordingly vary from country to country.

In the following Examples, which illustrate the invention, % and parts are by weight unless stated otherwise and temperatures in centigrades.

## EXAMPLE 1 - Selected whey protein hydrolysate

### 1.1 Preparation of Protein Solution

In a tank of 12'000 are introduced 7500 l demineralised water at a temperature of $25 \pm 2°C$.

900 kg of delipidated delactosed lactoserum are gradually dissolved therein. The mixture is rinsed with 600 l of demineralised water having a temperature of $25 \pm 2°$ and the volume adjusted to 11 500 l.

The hydration time is determined at 1 hour.

### 1.2 Thermic Treatment

The pH of the solution is then adjusted at $4.6 \pm 0.1$ employing a prepared mixture of citric acid and lactic acid (Mixture A; see below, Ex. 2.1).

The mixture is pasteurized, at pH 4.6 by flash heat treatment at 80° C during 1 minute.

The temperature of the mixture is then increased to $43 \pm 2°$ C and the pH adjusted to pH $2.6 \pm 0.1$ employing a 33 % hydrochloric acid solution.

### 1.3 Pepsine hydrolysis

0.2 g bovine pepsine (BOVIPEP; Lab. Presure Granday) / kg protein are then added and the reaction vessel shuttled during 1 hour while maintaining the temperature at 43°C.

### 1.4 Demineralisation by Ultrafiltration

The pH is adjusted to pH $8 \pm 0.1$ employing a preprepared alkaline mixture B (see below). The temperature is maintained at $43 \pm 2°$ C.

The mixture is ultrafiltrated (on IRIS 3038 membranes - Rhône Poulenc; membrane surface 80 m$^2$, temperature 50°C) till obtention of 2500 l of permeate. At this stage, the diafiltration is started; 4800 l of permeate are eliminated while maintaining the volume of the retentate constant by addition, at the same debit 4800 l of demineralised water at $45 \pm 2°$ C.

The chloride content of the retentate is demineralised. It should not exceed 1.7 g/l. If it does, the ultrafiltration must be pursued by elimination of a supplementary volume of permeate till the chloride concentration has reached the desired level.

The retentate volume is adjusted to 12000 l employing demineralised water having a temperature of $45 \pm 2°$ C.

The temperature and pH is verified; if necessary the temperature is readjusted by heating to $45 \pm 2°$ C and the pH re-adjusted to pH 8 by addition of alkaline mixture B (Ex. 2.2 below).

1.5 Trypsin/Chymotrypsin Hydrolysis in Presence of Elastase

PEM (Ex. 2.3 hereinafter) and elastase (Ex. 2.4 hereinafter) are added simultaneously in the form of prepared solutions: PEM at a rate of 3.5 g enzyme per kg of protein substrate and elastase at 1120 $U_2$ and max. 3800 $U_1$ per kg of protein substrate.

The pH is maintained at pH $8 \pm 0.1$ during 1 h 15 employing alkaline neutralization solution B prepared in advance.

The hydrolysis is continued for a further 1 h 15 period without further pH adjustment. During the total PEM/elastase hydrolysis period, agitation of the mixture is continued and the temperature maintained at $45 \pm 2°$ C.

### 1.6 Ultrafiltration/Diafiltration - Concentration

The protein hydrolysate solution is then subjected to ultrafiltration at 50° C, employing IRIS 3038 membranes (Rhône Poulence) - membrane surfaces 80 m².

The ultrafiltration is followed by a diafiltration when the refraction index of the retentate attains a value of ca. 13 %.

The diafiltration degree employed is 1.5.

The permeate is then cooled and concentrated till it has a refraction index of 35-40 %.

Residual proteins are removed by ultrafiltration on IRIS 3038 membranes (Rhône Poulenc), having a membrane surface of 80 m², at 50° C.

### 1.7 Sterilization / Drying

The concentrated permeate is sterilized (5 minutes at 130° C) and then spray dried (inlet temperature 180° C; outlet temperature 80° C).

The thus obtained product has the following physiochemical characteristics:

| | |
|---|---|
| solubility | 100 % |
| pH | 6 % |
| dry extract | 97.75 g/100 g |
| Proteins (N x 6.5) | 89.34 g/100 g |
| Total nitrogen (TN) | 13.74 g/100 g |
| Ashes | 7.76 g/100 g |
| | |
| Ca | 270 mg/100 g |
| Na | 580 mg/100 g |
| K | 2300 mg/100 g |
| Cl | 2860 mg/100 g |
| | |
| N in free amino groups (AN) | 2.66 g/100g |
| Total N content (NH$_2$ or NH) (TAN) | 12.80 g/100 g. |
| | |
| Apparent degree of hydrolysis (AN/TN) | 19.35 % |
| Effective degree of hydrolysis (AN/TAN) | 20.75 %. |

| MW repartition (determined by UV detection at 215 nm) | |
|---|---|
| MW > 5000 | 3.3 % |
| 5000 > MW > 3000 | 5.2 % |
| 3000 > MW > 1000 | 33.9 % |
| 1000 > MW > 500 | 30.9 % |
| 500 > MW > 300 | 20.6 % |
| 300 > MW | 6.1 % |

The aminogram (g/100 amino acids) of the thus obtained hydrolysate is as follows (average of 6 samples):

| Lys | $10.72 \pm 0.47$ | Ala | $5.24 \pm 0.29$ |
|---|---|---|---|
| His | $2.02 \pm 0.14$ | Cys/Cystine | $2.96 \pm 0.76$ |
| Arg | $2.58 \pm 0.25$ | Val | $4.23 \pm 0.23$ |
| Asp | $12.06 \pm 0.66$ | Met | $1.66 \pm 0.09$ |
| Thr | $4.67 \pm 0.22$ | Ile | $4.65 \pm 0.66$ |
| Ser | $3.74 \pm 0.24$ | Leu | $11.94 \pm 0.67$ |
| Glu | $18.47 \pm 0.69$ | Tyr | $3.04 \pm 0.10$ |
| Pro | $4.68 \pm 0.55$ | Phe | $3.63 \pm 0.29$ |
| Gly | $1.72 \pm 0.33$ | Trp | $2.24 \pm 0.29$ |

Free amino acid content (g/100 g product) - average of 3 samples:

| Lys | $0.72 \pm 0.09$ | Gly | $0.00 \pm 0.00$ |
|---|---|---|---|
| His | $0.01 \pm 0.02$ | Ala | $0.00 \pm 0.00$ |
| Arg | $0.15 \pm 0.00$ | Cys/Cystine | $0.00 \pm 0.00$ |
| Asp | $0.00 \pm 0.00$ | Val | $0.00 \pm 0.00$ |
| Asn | $0.00 \pm 0.00$ | Met | $0.01 \pm 0.01$ |
| Thr | $0.00 \pm 0.00$ | Ile | $0.05 \pm 0.02$ |
| Ser | $0.01 \pm 0.01$ | Leu | $0.26 \pm 0.07$ |
| Glu | $0.00 \pm 0.00$ | Tyr | $0.19 \pm 0.11$ |
| Gln | $0.00 \pm 0.00$ | Phe | $0.49 \pm 0.17$ |
| Pro | $0.00 \pm 0.00$ | Trp | $0.00 \pm 0.00$ |
| | | **TOTAL** | $\mathbf{1.89 \pm 0.42}$ |

Peptide composition (average of 6 samples):

| Detection UV (215 nm) | Peptides with 2-5 amino acids: | $28.80 \pm 0.65$ |
| | Peptides with 2-10 amino acids: | $60.83 \pm 0.87$ |
| | Peptides with 4-10 amino acids: | $39.52 \pm 1.19$ |

| Detection Refr. Index | Peptides with 2-5 amino acids: | $27.42 \pm 3.78$ |
| | Peptides with 2-10 amino acids: | $64.77 \pm 1.92$ |
| | Peptides with 4-10 amino acids: | $47.20 \pm 2.67$ |

## EXAMPLE 2

### 2.1 Mixture A

To 60 l demineralised water at 25 + 2° C are added 30 kg crystalline citric acid, while stirring.

After complete dissolution of the citric acid, 30 l lactic acid (purity 80 %) are added. Then further demineralised water is added till a total volume of 130 l is obtained.

### 2.2 Mixture B

To 100 l demineralised water at $25 \pm 2°$ C are added 52.97 kg KOH while stirring, till complete dissolution. Then 211.75 l pure 21 % ammonia are added and the volume adjusted to 350 l by addition of demineralised water.

### 2.3 PEM (3.5 g enzyme/kg of proteins)

The Proteolytic Enzyme Mixture employed is a concentrated mixture in powder form without salts, of porcine trypsin, bovine trypsin and bovine chymotrypsin, commercial designation P.E.M. 2500 S (NOVO Industrie Enzymes S.A. Paris), having a trypsine activity of at least 1800 USP-u/mg and a chymotrypsine activity of at least 350 USP-u/mg.

It is employed as an aqueous solution, by dissolution of PEM 2500 S in demineralised water at $25 \pm 2°$ C.

### 2.4 Elastase (Pancreopeptidase E)

The elastase employed is from porcine pancreas origin, commercialised under Code E 3 by Biozyme (GB), in the form of a suspension in 70 % saturated ammonium sulphate with an activity of not less than 120 U/mg protein.

The unit definition employed by Biozyme is as follows: the amount of enzyme causing the hydrolysis of 1 micromole of N-acetyl-tri-L-alanine methyl ester per minute at 25° C and pH 8.5 (Method of assay: Gertler and Hofmann (1970) Can. J. Biochem. 48 384).

## EXAMPLE 3 - Whey Protein Hydrolysate

One proceeds analogous to the procedure of Example 1, except that after the neutralization phase of 1h 15 (step 1.5) the hydrolysis is continued for another 1h 45 (instead of 1h 15 minutes).

The MW repartition is as follows: (determined by UV detection at 215 nm)

| MW > 5000 | 3.4 |
|---|---|
| 5000 > MW > 3000 | 10.0 |
| 3000 > MW > 1000 | 34.9 |
| 1000 > MW > 500 | 28.9 |
| 500 > MW > 300 | 15.4 |
| 300 > MW | 7.4 |

## EXAMPLE 4 - Whey Protein Hydrolysate

One proceeds analogous to the procedure of Example 1, except that after the neutralization phase of 1h 15 (step 1.5), 1 % COROLASE 7092 is added and the hydrolysis continued for 1h 15.

COROLASE 7092 (Röhm) is a proteolytic enzyme preparation in liquid form from Aspergillus oryzae cultures having an activity of 2000 m UHb/mg, 1UHb being the enzyme quantity releasing with one minute at pH 7.5 and 37° C the equivalent of 1 micro mole tyrosine in TCE-soluble hydrolysate from haemoglobulin.

The thus obtained product has the following physiochemical characteristics:

| Dry Extract | 97.97 g/100 g |
|---|---|
| Total nitrogen content (kjeldahl) | 13.30 g/100 g |
| Protein content (x 6.5) | 85.01 g/100 g |
| Ashes | 7.99 g/100 g |
| Calcium | 0.33 g/100 g |
| Sodium | 0.67 g/100 g |
| Potassium | 3.05 g/100 g |
| Chlorides | 2.72 g/100 g |
| pH | 6.05 |
| Solubility | 100 % |

Its MW repartition is as follows: (determined by UV detection at 215 nm)

| MW > 5000 | 0.7 |
|---|---|
| 5000 > MW > 3000 | 2.0 |
| 3000 > MW > 1000 | 29.3 |
| 1000 > MW > 500 | 26.0 |
| 500 > MW > 300 | 29.3 |
| 300 > MW | 12.7 |

## EXAMPLE 5 - Whey Protein hydrolysate

One proceeds analogous to the procedure of Example 4, except that 1 % COROLASE PP, a pancreatic proteinase, is employed instead of COROLASE 7092.

## EXAMPLE 6 - Whey Protein hydrolysate

One proceeds analogous to the procedure of Examples 1 to 4, except that the whey protein employed as a starting material is as follows pretreated to remove large proteins.

Commercially available delactosed lactoserum protein powder is purified and pretreated to remove macrolipids and most of large proteins having a molecular weight of more than 60'000 Daltons employing microfiltration and ultrafiltration techniques on membranes having a dynamic cut-off over 50'000.

The conditions are selected such that ca. 95 % of the immunoglobulins IgG are eliminated by microfiltration on membrane 0.22 micron, measured by immunotechnic and that the product comprises ca. 75 g proteins per litre and less than 2 g of lactose per litre.

## EXAMPLE 7 - Trypsin/Chymotrypsin/Elastase-type 2 - hydrolysis of rennet casein

a) 1000 kg of rennet casein (obtained from milk by enzymic precipitation with rennet, comprising at least 84 % by weight of proteins-relative to the total dry material and having a water content of 10 % or less) are added portionwise with stirring to a reaction vessel of 12 $m^3$ comprising 7000 l of demineralised water, cooled at a temperature of 5° C. The mixture is rinsed with 700 l of demineralised water and the volume of the mixture adjusted to 8300 l. The hydration time is 1 hour.

b) The pH of the solution of step a) is adjusted to pH $8 \pm 0.1$ employing the alkaline solution [B] of ammonia and potassium hydroxide (according to Example 2.2).

c) The temperature is adjusted to 45° C $\pm$ 2° C.

d) PEM (see Ex. 2.3) and elastase (see Ex. 2.4) are added simultaneously to the thus obtained mixture: PEM at a rate of 1.96 g/kg of proteins, elastase at a rate of 3420 $U_1$ per kg of protein.

The pH is maintained at $8 \pm 0.1$ for 1 h 15 by neutralization of the solution employing the ammonia/potassium hydroxide solution B (Example 2.2).

e) COROLASE 7092 (Rhöm) is then added at a rate of 15 g/kg proteins. The mixture is kept at 45° $\pm$ 2° C for 1hr 15.

f) After the hydrolysis has been finalized (the total hydrolysis duration is 2h 30) the mixture is subjected to ultrafiltration employing IRIS 3038 membranes (Rhone-Poulenc) having a membrane surface of 80 $m^2$. The temperature is 50° C.

g) The ultrafiltration is followed by a diafiltration when the refraction index attains a value of about 13 %.

The diafiltration degree employed is 1.5.

The temperature of the permeate must be cooled to below 5° C if it is stored for more than 2 hours.

h) The permeate is concentrated by evaporation till the refraction index value is 35 to 40 %.

The concentrate is sterilized at 125° $\pm$ 2° C for 1 minute and then spray dried (inlet temp. 180° C; outlet temp. 80° C).

The thus obtained casein hydrolysate has the following characteristics:

| | |
|---|---|
| Solubility | 100 % |
| pH | 7.26 |
| Dry extract | 97.66 g/100 g |
| Proteins (N x 6.5) | 94.12 g/100 g |
| Total N content (TN) | 14.45 g/100 g |
| Ashes | 4.27 g/100 g |
| | |
| Calcium | 370 mg/100 g |
| Na | 50 mg/100 g |
| K | 1240 mg/100 g |
| Cl | 90 mg/100 g. |
| | |
| N in free amino groups (AN) | 3.35 g/100 g |
| Total N content (NH or $NH_2$) (TAN) | 12.40 g/100 g |
| | |
| Apparent degree of hydrolysis (AN/TN) | 23.2 % |
| Effective degree of hydrolysis (AN/TAN) | 27.0 % |

| Repartition of MW: (determination by UV detection at 215 nm) | |
|---|---|
| MW > 5000 | 0 % |
| 5000 > MW > 3000 | 0.1 % |
| 3000 > MW > 1000 | 18.6 % |
| 1000 > MW > 500 | 37.4 % |
| 500 > MW > 300 | 29.2 % |
| 300 > MW | 14.7 % |

Free amino acid content 10.5 %.

| Aminogram (in g/100 g Amino acids) | | | | | | | |
|---|---|---|---|---|---|---|---|
| Lys | 7.9 | Ala | 2.7 | His | 3.0 | Cys | 0.5 |
| Arg | 3.8 | Val | 5.7 | | | | |
| Asp | 6.7 | Met | 2.7 | | | | |
| Thr | 3.3 | Ile | 3.8 | | | | |
| Ser | 3.9 | Leu | 9.8 | | | | |
| Glu | 20.7 | Tyr | 6.1 | | | | |
| Pro | 10.7 | Phe | 5.6 | | | | |
| Gly | 1.9 | Trp | 1.4 | | | | |

| Osmolarity mosmol/l | |
|---|---|
| 50 g/l | 119 |
| 100 g/l | 236 |

Residual allergenicity: none

**EXAMPLE 8 - Formulation for infants**

| | |
|---|---|
| Hydrolysed proteins * | 13.1 g |
| Fat | 20.2 g |
| Carbohydrates | 58.4 g |
| Minerals | 2.8 g |
| Non caloric organic substances** | 0.7 g |
| Humidity | 4.8 g |
| TOTAL | 100 g |

* (the 60:40 whey protein: casein mixture of which the whey protein is hydrolysed according to Example 1 after pretreatment according to Example 6 and the rennet casein is hydrolysed according to Example 7). The hydrolysate is supplemented

with 0.055 g L-cystine and 0.03 g of taurine per 100 g finished product.

** refers to the vitamin fraction and to the organic ligand component of certain trace elements and minerals of the formulation

Its aminogram is as follows (in g/100 g amino acid)

| Asp/Asn | 9.43 | Met | 2.24 |
|---------|------|-----|------|
| Thr | 4.59 | Ile | 5.33 |
| Ser | 4.17 | Leu | 11.80 |
| Glu/Gln | 18.65 | Tyr | 2.95 |
| Pro | 7.84 | Phe | 4.47 |
| Gly | 1.68 | Lys | 9.78 |
| Ala | 4.31 | His | 2.51 |
| Cystine | 0.71 | Arg | 2.90 |
| Val | 5.48 | Trp | 1.16 |

## Claims

1. The use of whey protein hydrolysate substantially free of allergenic proteins for the manufacture of a dietary composition for the induction of cow's milk tolerance in children susceptible to cow's milk allergy.

2. The use according to Claim 1, wherein the whey protein hydrolysate is obtainable by hydrolysis of whey protein with trypsin/chymotrypsin in the presence of elastase 2.

3. The use according to Claim 1, wherein the whey protein hydrolysate is obtainable by prehydrolysis with pepsin, followed by hydrolysis of the whey protein prehydrolysate with trypsin/chymotrypsin in the presence of elastase 2.

4. The use according to Claim 1, wherein the whey protein hydrolysate is obtainable by prehydrolysis with pepsin, followed by hydrolysis of the whey protein prehydrolysate with trypsin/chymotrypsin in the presence of elastase 2 and - after an initial pancreatic hydrolysis phase - in the presence of the fungal proteolytic enzyme from Aspergillus oryzae having endo- and exopeptidase activity.

5. The use according to Claim 1, wherein the whey protein hydrolysate is obtainable by prehydrolysis with pepsin, followed by hydrolysis of the whey protein prehydrolysate with trypsin/chymotrypsin in the presence of elastase and - after an initial hydrolysis phase - in the presence of pancreatic proteinase extract.

6. The use according to Claims 2 to 5, wherein the whey protein employed as starting material is substantially free of macrolipids.

7. The use according to Claims 2 to 6, wherein the whey protein employed as starting material is substantially free of lactose.

8. The use according to Claims 2 to 7, wherein the whey protein employed as starting material is substantially free of proteins having a molecular weight of more than 60'000 Daltons.

9. The use according to Claims 1 to 8, wherein the dietary composition comprises as an additional protein source casein hydrolysate substantially free of allergenic proteins.

10. The use according to Claim 9, wherein the casein hydrolysate is obtainable by hydrolysis of casein with trypsin/chymotrypsin in the presence of elastase 2.

11. The use according to Claims 9 or 10, wherein the casein employed as starting material is substantially free of glycoprotein.

12. The use according to Claims 9 to 11, wherein the casein employed as starting material is rennet casein.

13. The use according to Claims 9 to 12, wherein the whey protein and casein hydrolysates are in a weight ratio whey protein hydrolysate: casein hydrolysate of 5:1 to 1:1.

14. The use according to Claims 1 to 13, wherein the dietary composition is in the form of a complete formula diet.

15. The use of whey protein hydrolysate substantially free of allergenic proteins for the manufacture of a dietary composition for the prophylaxis or treatment of type 1 diabetes mellitus in children susceptible to such disease.

16. The use according to Claim 15, wherein the whey protein hydrolysate is obtainable by hydrolysis of whey protein with trypsin/chymotrypsin in the presence of elastase 2.

17. The use according to Claim 15, wherein the whey protein hydrolysate is obtainable by prehydrolysis with pepsin, followed by hydrolysis of the whey protein prehydrolysate with trypsin/chymotrypsin in the presence of elastase 2.

18. The use according to Claim 15, wherein the whey protein hydrolysate is obtainable by prehydrolysis with pepsin, followed by hydrolysis of the whey protein prehydrolysate with trypsin/chymotrypsin in the presence of elastase 2 and - after an initial pancreatic hydrolysis phase - in the presence of the fungal proteolytic enzyme from Aspergillus oryzae having endo- and exopeptidase activity.

19. The use according to Claim 15, wherein the whey protein hydrolysate is obtainable by prehydrolysis with pepsin, followed by hydrolysis of the whey protein prehydrolysate with trypsin/chymotrypsin in the presence of elastase and - after an initial hydrolysis phase - in the presence of pancreatic proteinase extract.

20. The use according to Claims 16 to 19, wherein the whey protein employed as starting material is substantially free of macrolipids.

21. The use according to Claims 16 to 20, wherein the whey protein employed as starting material is substantially free of lactose.

22. The use according to Claims 16 to 21, wherein the whey protein employed as starting material is substantially free of proteins having a molecular weight of more than 60'000 Daltons.

23. The use according to Claims 15 to 22, wherein the dietary composition comprises as an additional protein source casein hydrolysate substantially free of allergenic proteins.

24. The use according to Claim 23, wherein the casein hydrolysate is obtainable by hydrolysis of casein with trypsin/chymotrypsin in the presence of elastase 2.

25. The use according to Claims 23 or 24, wherein the casein employed as starting material is substantially free of glycoprotein.

26. The use according to Claims 23 to 25, wherein the casein employed as starting material is rennet casein.

27. The use according to Claims 23 to 26, wherein the whey protein and casein hydrolysates are in a weight ratio whey protein hydrolysate: casein hydrolysate of 5:1 to 1:1.

28. The use according to Claims 15 to 27, wherein the dietary composition is in the form of a complete formula diet.

Figure 1

Fig. 1a

Fig. 1b

Fig. 1c

Fig. 1d

Figure 2

Fig. 2a

Fig. 2b

**European Patent Office**

# EUROPEAN SEARCH REPORT

Application Number

EP 94 81 0352

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | DATABASE WPI Week 9305, Derwent Publications Ltd., London, GB; AN 93-040474 & JP-A-4 365 444 (MORINAGA MILK IND. CO. LTD.) 17 December 1992 | 1 | A23J3/34 A23L1/305 |
| Y | * abstract * | 2-14 | |
| X | DATABASE WPI Week 9227, Derwent Publications Ltd., London, GB; AN 92-223434 & JP-A-4 149 139 (NIPPON STEEL CORP.) 22 May 1992 | 15 | |
| Y | * abstract * | 16-28 | |
| D,Y | EP-A-0 421 309 (SANDOZ NUTRITION LTD.) * claims 1,6,9,13,22,24,30; examples 6,7,10,14 * * page 5, line 21 - page 6, line 11; examples 18,20,24 * | 2-14, 16-28 | |
| | | | **TECHNICAL FIELDS SEARCHED (Int.Cl.5)** |
| A | PATENT ABSTRACTS OF JAPAN vol. 017, no. 266 (C-1062) 25 May 1993 & JP-A-05 005 000 (RIYOUSHIYOKU KENKIYUUKAI) 14 January 1993 * abstract * & DATABASE WPI Week 9307, Derwent Publications Ltd., London, GB; AN 93-055215 & JP-A-5 005 000 (RYOSHOKU KENKYUKAI) 14 January 1993 * abstract * | 1,2,8 | A23J A23L A61K |

-/--

The present search report has been drawn up for all claims

| Place of search | Date of completion of the search | Examiner |
|---|---|---|
| THE HAGUE | 23 September 1994 | Kanbier, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EP 0 629 350 A1

European Patent Office

# EUROPEAN SEARCH REPORT

Application Number

EP 94 81 0352

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | ANN. MED., vol.23, no.4, 1991, HELSINKI pages 447 - 452 J.M. MARTIN ET AL 'MILK PROTEINS IN THE ETIOLOGY OF INSULIN-DEPENDENT DIABETES MELLITUS (IDDM)' --- | 1,15 | |
| A | CHEMICAL ABSTRACTS, vol. 79, no. 19, 12 November 1973, Columbus, Ohio, US; abstract no. 114211, 'REMOVAL OF BITTER TASTE FROM PEPTIDE SOLUTION OBTAINED FROM MILK CASEIN AND WHEY' * abstract * & DATABASE WPI Week 7625, Derwent Publications Ltd., London, GB; AN 73-66177 & JP-A-48 052 967 (KIKKOMAN SHOYU CO. LTD.) 1973 * abstract * --- | 4,5,18, 19 | |
| A | WO-A-87 03785 (O.M. POULSEN) * page 6, line 26 - page 7, line 19; claims 1,10; example 2 * ----- | 5,19 | **TECHNICAL FIELDS SEARCHED** (Int.Cl.5) |

The present search report has been drawn up for all claims

| Place of search | Date of completion of the search | Examiner |
|---|---|---|
| THE HAGUE | 23 September 1994 | Kanbier, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

20